(19) Europäisches Patentamt
European Patent Office
Office européen des brevets

(11) Numéro de publication: **0 196 938**
**A1**

(12) **DEMANDE DE BREVET EUROPEEN**

(21) Numéro de dépôt: **86400422.1**

(22) Date de dépôt: **27.02.86**

(51) Int. Cl.4: **C08F 220/28**, C08F 220/34, B01J 20/26, C07K 17/08

(30) Priorité: **05.03.85 FR 8503205**

(43) Date de publication de la demande: **08.10.86 Bulletin 86/41**

(84) Etats contractants désignés: **BE DE GB IT NL SE**

(71) Demandeur: **COMMISSARIAT A L'ENERGIE ATOMIQUE Etablissement de Caractère Scientifique Technique et Industriel**
**31/33, rue de la Fédération**
**F-75015 Paris(FR)**

(72) Inventeur: **Colin, Claude**
**3, rue de Bretagne Val des 4 Pignons**
**F-78650 Beynes(FR)**
Inventeur: **Duval, Dominique**
**11, rue P. Brossolette 333G**
**F-92400 Courbevoie(FR)**
Inventeur: **Gaussens, Gilbert**
**11, rue Jean Brunet**
**F-92190 Meudon(FR)**
Inventeur: **Morillon, Cécile**
**99, rue de Paris**
**F-91400 Orsay(FR)**
Inventeur: **Nicaise, Maryvonne**
**38, Avenue des Bois**
**F-78470 Saint Remy Les Chevreuse(FR)**
Inventeur: **Vergne, Gérard**
**38, rue Albert Camus**
**F-92160 Antony(FR)**

(74) Mandataire: **Mongrédien, André et al**
**c/o BREVATOME 25, rue de Ponthieu**
**F-75008 Paris(FR)**

(54) **Support solide capable de fixer une substance biologique, son procédé de préparation et son utilisation pour l'épuration plasmatique.**

(57) L'invention a pour objet un support solide capable de fixer une substance biologique, son procédé de préparation et son utilisation pour l'épuration plasmatique.

Ce support est constitué par un copolymère :

-d'au moins un monomère choisi parmi les monomères acryliques, les monomères méthacryliques et les monomères allyliques, porteurs d'au moins une fonction chimique réactive présentant une affinité pour la substance biologique à fixer, et

-d'au moins un monomère réticulant comportant au moins deux fonctions éthyléniques réactives sous rayonnements ionisants.

Ce copolymère peut être préparé en soumettant à une irradiation au moyen de rayonnements ionisants un mélange de deux types de monomères éventuellement en solution dans un solvant.

Un copolymère de méthacrylate de diméthylaminoéthyle et de triméthacrylate de triméthylol propane sur lequel sont fixées des hématies peut être utilisé dans la colonne 7 pour épurer du plasma sanguin en anticorps anti-D humain.

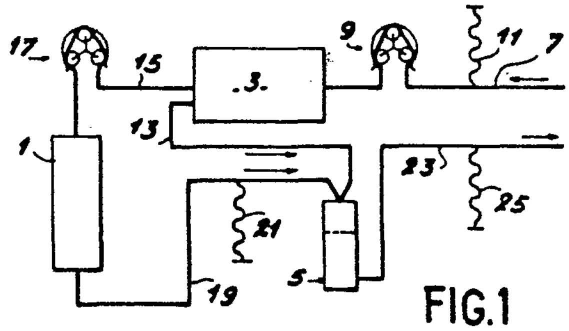


FIG.1

EP 0 196 938 A1

# Support solide capable de fixer une substance biologique, son procédé de préparation et son utilisation pour l'épuration plasmatique.

La présente invention a pour objet un support solide insoluble capable de fixer de façon irréversible une substance biologique, son procédé de préparation par irradiation au moyen de rayonnements ionisants et son utilisation pour l'épuration plasmatique.

De façon plus précise, elle concerne la réalisation de supports polymérisés comportant des fonctions chimiques capables de retenir sélectivement une substance biologique. De tels supports sur lesquels sont fixées de façon irréversible des substances biologiques, peuvent être utilisés comme adsorbants pour la chromatographie d'affinité ou pour la récupération ou l'élimination de substances biologiques présentes dans le sang, ce qui les rend utiles pour l'épuration plasmatique.

L'épuration plasmatique est une technique de traitement relativement récente qui vise des objectifs thérapeutiques variés. Cette technique consiste à épurer le sang d'un patient afin d'extraire de celui-ci des produits indésirables tels que des anticorps dans le cas de malades souffrant de maladies autoimmunes ou de problèmes dûs à une incompatibilité sanguine ou à des réactions de rejet.

Jusqu'à présent, pour supprimer ces éléments indésirables, on utilisait les techniques de plasmaphérèse qui consistent à substituer en partie le plasma des malades par un plasma étranger ou par des solutions de remplacement. Cependant, ces techniques ne sont pas satisfaisantes, car elles nécessitent l'injection d'un plasma étranger et elles ne permettent pas de maintenir aux taux voulus les éléments plasmatiques constitutifs indispensables. Aussi, les procédés d'épuration directe du plasma sanguin ont fait récemment l'objet de nombreuses recherches, et on a envisagé d'utiliser dans ce but des phases solides porteuses d'éléments possédant une affinité spécifique pour l'élément à épurer par exemple des supports solides pour chromatographie d'affinité tels que la Sépharose et la silice. Cependant, ces supports ne possèdent pas l'ensemble des caractéristiques requises pour une épuration "ex vivo". En effet, leurs caractéristiques mécaniques sont insuffisantes ; par ailleurs, la géométrie et la granulométrie de ces supports solides ne sont pas adaptées à l'hémodynamique et ils n'ont pas des propriétés suffisantes de biocompatibilité et de biorésistance.

Aussi, on s'est orienté plus récemment sur l'utilisation de supports solides constitués par des polymères porteurs d'éléments biofonctionnels, fixés par des liaisons covalentes sur ces supports. La réalisation de tels supports est décrite en particulier dans le brevet français FR-A-2 534 486 au nom du Commissariat à l'Energie Atomique.

Dans ce brevet, on utilise un support inerte constitué par des particules d'une matière polymère de base permettant de conférer au support les propriétés mécaniques voulues, en particulier la tenue dimensionnelle sous pression, la résistance à l'écrasement, la densité, une bonne inertie physique et physicochimique, l'insolubilité dans la plupart des solvants et un taux de gonflement faible. On greffe ensuite sur ce polymère de base un monomère insaturé porteur d'au moins une fonction chimique capable de former ultérieurement une liaison covalente avec un ligand spécifique de la substance à éliminer ou à extraire. Ce greffage permet ainsi de conférer au polymère de base inerte la faculté de fixer un ligand spécifique de la substance à éliminer ou à extraire.

Bien que de tels supports soient satisfaisants, ils présentent l'inconvénient de nécessiter l'utilisation d'un polymère de base sous forme particulaire que l'on soumet ensuite à un greffage, ce qui limite le nombre de sites actifs accessibles pour la fixation du Ligand.

On connaît d'autres supports à base de copolymère qui peuvent être utilisés en biologie, soit comme phase stationnaire pour la séparation de substances biologiques telles que les protéines, les enzymes et les aminoacides, par chromatographie liquide, soit pour isoler et caractériser des membranes cellulaires, comme il est décrit dans la demande de brevet européen EP-A-0 063 947 et dans le brevet américain US-A-4 105 598.

Dans la demande de brevet européen EP-A-0 063 947, le copolymère est formé à partir d'un mélange de monomères comprenant :

a) -un monomère polymérisable ayant au moins une double liaison polymérisable et au moins un groupe carboxyle par molécule,

b) -un monomère réticulant contenant au moins deux double liaisons polymérisables par molécule et éventuellement,

c) -un monomère additionnel ayant au moins une double liaison par molécule.

Selon ce brevet, il est important que le copolymère ne retienne pas de façon irréversible la substance biologique à fixer afin de pouvoir être utilisé en séparation par chromatographie liquide. Pour obtenir ce résultat, on choisit des monomères présentant un paramètre d'hydrophobie d'au plus 2,3, et de préférence d'au plus 0. De tels copolymères ne peuvent donc être utilisés pour fixer de façon irréversible une substance biologique comme on le recherche dans l'invention.

Dans le brevet américain US-A-4 105 598, on utilise un copolymère comportant trois types de monomères dont un premier monomère acrylique éthyléniquement insaturé contenant au moins une fonction hydroxyle, carboxyle ou amino, un second monomère éthyléniquement insaturé ayant une densité différente de celle du premier monomère et un agent réticulant polyinsaturé. La présence du deuxième monomère permet de régler la densité des particules de copolymère obtenues sur lesquelles on peut fixer des molécules biologiques telles que des anticorps, des lectines, des hormones et des toxines, en vue d'utiliser ces particules pour l'identification de cellules particulières ou pour la détection et la localisation de récepteurs de surface de cellules spécifiques.

De tels copolymères ont l'inconvénient de nécessiter la présence d'un troisième monomère pour obtenir la densité souhaitée, ce qui limite la teneur en réticulant du copolymère et ne permet pas l'obtention de caractéristiques satisfaisantes pour une utilisation en épuration plasmatique.

La présente invention a précisément pour objet un support polymérisé qui pallie les inconvénients précités, ne nécessite pas l'utilisation d'une matière polymère de base inerte en particules, et peut être fabriqué de façon simple tout en conduisant à un produit présentant un nombre suffisant de sites actifs accessibles pour la fixation d'un ligand, et une structure qui peut être adaptée facilement à l'utilisation envisagée.

Selon l'invention, le support solide capable de fixer de façon irréversible une substance biologique, se caractérise en ce qu'il est constitué par un copolymère :

-d'au moins un monomère choisi parmi les monomères acryliques, les monomères méthacryliques et les monomères allyliques, porteurs d'au moins une fonction chimique réactive présentant une affinité pour la substance biologique à fixer, choisie parmi les fonctions amine primaire, amine secondaire, amine tertiaire, hydroxyle, aldéhyde et amide, et

-d'au moins un monomère réticulant comportant au moins deux fonctions éthyléniques réactives sous rayonnements ionisants.

Le support polymérisé de l'invention présente en particulier l'avantage de ne pas nécessiter le greffage d'un monomère réactif sur un polymère de base inerte. En effet, en choisissent de façon appropriée les conditions de fabrication du copolymère et les taux de monomères réticulants et de monomères porteurs de fonctions chimiques réactives ayant une affinité pour la substance biologique à fixer, on peut obtenir les propriétés mécaniques voulues pour l'épuration plasmatique, en particulier les qualités de tenue dimensionnelle sous pression, de résistance à l'écrasement, de densité, d'inertie chimique et physicochimique, d'insolubilité dans la plupart des solvants et de taux de gonflement faible. Par ailleurs, on peut obtenir la quantité voulue de sites réactifs, car ceux-ci sont beaucoup plus accessibles que dans le cas d'un polymère de base greffé par un monomère réactif.

Généralement, le support comprend :

-30 à 80% en poids dudit ou desdits monomères porteurs d'au moins une fonction chimique réactive présentant une affinité pour la substance biologique à fixer, et

-de 20 à 70% en poids dudit ou desdits monomères réticulants.

Les différents monomères entrant dans la composition du copolymère sont choisis en particulier en fonction de l'utilisation du support et des substances biologiques à fixer sur ce dernier.

On précise que l'on entend par "substance biologique" toute molécule ayant une activité biologique. A titre d'exemples de telles substances, on peut citer les anticorps, en particulier les anticorps monoclonaux, les lectines, les cellules par exemple les hématies, les antigènes, les haptènes, les hormones, les fragments et combinaisons AC-fragments, les drogues, les récepteurs, les stéroïdes, les aminoacides, les peptides, les substrats et les inhibiteurs d'enzymes.

L'un des monomères utilisés pour la fabrication du copolymère doit comporter au moins une fonction chimique réactive présentant une affinité pour la substance biologique à fixer sur le support. Cette affinité peut être obtenue dans la plupart des cas lorsque l'on utilise un monomère insaturé comportant une fonction choisie parmi les fonctions amine primaire, amine secondaire, amine tertiaire, hydroxyle, aldéhyde et amide. Dans le cas de monomères comportant une fonction amine tertiaire, on obtient une affinité ionique très élevée du copolymère pour la plupart des substances biologiques à fixer sur le support.

A titre d'exemple de monomères insaturés comportant des fonctions chimiques réactives, susceptibles d'être utilisés dans l'invention, on peut citer les acrylates et méthacrylates de dialkylaminoalkyle, par exemple le méthacrylate de diméthylaminoéthyle, l'acroléine, la méthacroléine, l'acrylamide, la méthacrylamide, l'allylamide, les acrylates et méthacrylates d'éthylène glycol et de polyéthylène glycol, par exemple l'acrylate d'éthylène glycol.

Le ou les monomères réticulants entrant dans la composition du copolymère sont choisis également en fonction de l'utilisation envisagée. Généralement, on utilise des monomères polyacryliques ou polyméthacryliques.

A titre d'exemples de monomères réticulants susceptibles d'être utilisés, on peut citer les polyacrylates et polyméthacrylates de polyols, par exemple le diacrylate d'éthylène glycol, le diacrylate de tétraéthylène glycol, le triacrylate de triméthylol propane, le diméthacrylate d'éthylène glycol, le diméthacrylate de tétraéthylène glycol, le triméthacrylate de triméthylol propane, le tétracrylate de pentaérythrol et le tétraméthacrylate de pentaérythrol.

Selon un mode préféré de réalisation de l'invention, le support est constitué par un copolymère de méthacrylate de diméthylaminoéthyle et de triméthacrylate de triméthylol propane.

L'invention a également pour objet un procédé de préparation du support précité.

Ce procédé consiste à polymériser au moyen de rayonnements ionisants un mélange de monomères comprenant au moins un monomère choisi parmi les monomères acryliques, méthacryliques et allyliques, porteurs d'au moins une fonction chimique présentant une affinité pour la substance biologique à fixer, et au moins un monomère réticulant comportant au moins deux fonctions éthyléniques réactives sous rayonnements ionisants.

Les rayonnements ionisants susceptibles d'être utilisés sont par exemple les rayonnements ultraviolets, les rayonnements X, les rayonnements $\alpha$, $\beta$ ou $\gamma$, et les faisceaux d'électrons accélérés. On utilise avantageusement les rayonnements $\gamma$ provenant d'une source de cobalt 60.

Le fait de réaliser, dans l'invention, la polymérisation des monomères au moyen de rayonnements ionisants est particulièrement avantageux car on peut ainsi contrôler et maîtriser la structure du copolymère obtenu, en particulier régler ses caractéristiques physico-chimiques par exemple sa densité, sa porosité ainsi que le nombre et la disposition des fonctions actives qu'il contient.

En revanche, si l'on utilisait les procédés classiques de copolymérisation par voie chimique, il serait plus difficile de contrôler et de maîtriser la structure du copolymère obtenu et ses propriétés physico-chimiques.

La polymérisation peut être réalisée en masse ou en émulsion.

Pour effectuer la polymérisation en masse, on soumet un mélange des deux types de monomères sus-mentionnés éventuellement dissous dans un solvant, et comportant les quantités voulues de monomères à fonction chimique réactive et de monomères réticulants, à une irradiation au moyen de rayonnements ionisants pour effectuer une polymérisation et une réticulation grâce à la présence des monomères réticulants capables de créer un réseau tridimensionnel.

Les solvants utilisés peuvent être des solvants organiques tels que le méthanol, l'éthanol ou l'acétone. On peut aussi utiliser des solvants aqueux, par exemple de l'eau.

Le copolymère obtenu dans ces conditions est ensuite soumis à un broyage et à un tamisage afin d'obtenir des particules de copolymère de dimensions appropriées.

Lorsque l'on veut réaliser la polymérisation en émulsion, on prépare tout d'abord une première phase constituée par le mélange de monomères en proportions voulues éventuellement dissous dans un solvant approprié,

puis on disperse cette première phase liquide sous forme d'émulsion dans une second phase liquide immiscible en mélangeant énergiquement les deux phases et on soumet ensuite l'émulsion à une irradiation au moyen de rayonnements ionisants. Généralement, la première phase est une phase organique et on la met en émulsion dans une phase aqueuse dont le pH est ajusté de façon à éviter le passage des monomères dans la phase aqueuse. On peut aussi ajouter à la phase aqueuse un agent inhibiteur de la polymérisation des monomères, par exemple du sel de Mohr, ainsi qu'un agent favorisant l'établissement de l'émulsion, par exemple de l'acide polyacrylique. Lorsque l'on utilise un solvant organique, celui-ci est choisi de façon à dissoudre les deux types de monomères tout en restant immiscible avec la seconde phase.

Dans les deux types de polymérisation, on règle la structure du copolymère obtenu en choisissant la proportion de monomère réticulant, la dose et le débit de dose d'irradiation appliqués au mélange de monomères.

Le débit de dose est avantageusement d'environ 0,01 à 0,25 Mrad/h (100 à 2500 Gy/h). La dose intégrée nécessaire pour réaliser la copolymérisation et la réticulation est généralement d'environ 0,9 à 8 Mrad (9000 à 80000 Gy).

Le taux de réticulation dépend en particulier de la valeur de la dose intégrée de rayonnements ionisants appliquée.

Ce mode de fabrication du support de l'invention présente de nombreux avantages qui sont liés, d'une part, à l'utilisation des rayonnements ionisants et, d'autre part, à la possibilité d'adapter la structure du copolymère à l'utilisation envisagée.

En effet, l'utilisation de rayonnements ionisants permet d'éviter l'adjonction de catalyseurs chimiques dont la présence pose des problèmes de biocompatibilité. Par ailleurs, la réaction de copolymérisation et de réticulation permet d'obtenir directement un support en particules utilisable pour la fixation de molécules biologiques, selon un mode opératoire simple qui conduit à un prix de revient avantageux du support.

De plus, en réglant de façon appropriée les proportions de monomères à fonction chimique réactive et de monomères réticulants, on peut adapter les propriétés mécaniques du support à l'utilisation envisagée et disposer de la quantité voulue de fonctions chimiques réactives accessibles.

Ainsi, on peut obtenir un support ayant des propriétés mécaniques équivalentes à celles des supports inertes utilisés précédemment et obtenir simultanément des propriétés physicochimiques améliorées pour la fixation de la substance biologique, ce qui conduit à une meilleure réactivité du support. De tels supports présentent en particulier des affinités ioniques spécifiques pour les substances biologiques choisies ou synthétiques. Ainsi, lorsque l'on met en contact le substrat avec une substance biologique, celle-ci est attirée sur le support et on peut la fixer ensuite sur ce support en créant des liaisons entre les molécules de la substance biologique, par exemple par réticulation au moyen d'un réactif approprié tel que le glutaraldéhyde ou le carbodiimide.

L'invention a donc également pour objet un procédé de préparation d'un support solide sous forme de particules sur lequel est fixée une substance biologique, qui consiste à mettre en contact des particules du support solide de l'invention avec une substance biologique en solution ou en suspension et à soumettre ensuite le support séparé de la solution ou de la suspension et ayant adsorbé la substance biologique à l'action d'un réactif capable de fixer ladite substance sur ledit support.

Après cette réaction de fixation, on soumet le support ainsi traité à différents rinçages effectués par exemple au moyen de sérum physiologique, d'une solution de glycine et/ou de solutions tampons.

La granulométrie des particules du support en copolymère est choisie en fonction de l'utilisation envisagée. Généralement la taille de particules se situe dans la gamme d'environ 10 à environ 5000 µm. Cette taille de particules peut être obtenue directement lorsque l'on réalise la polymérisation en émulsion en réglant de façon appropriée les proportions des deux phases et la vitesse d'agitation. Lorsque l'on réalise la polymérisation en masse, on soumet le copolymère obtenu à un broyage puis à un tamisage pour isoler la fraction de particules ayant la granulométrie appropriée.

L'invention a aussi pour objet un support contenant une substance biologique, constituée par des particules du support en copolymère de l'invention sur lesquels est fixée une substance biologique.

L'invention a de plus pour objet l'utilisation d'un tel support, en particulier dans le domaine de la biologie, et notamment pour extraire ou éliminer un élément biologique du sang, "in vitro" ou par circulation extracorporelle "ex vivo".

Pour ces différentes applications, on fixe sur le support de l'invention une substance biologique capable de réagir avec l'élément biologique à extraire ou à éliminer du sang.

A titre d'exemple, on peut utiliser le support de l'invention pour extraire ou éliminer un antigène du sang après avoir fixé sur ce support l'anticorps correspondant, en particulier l'anticorps monoclonal correspondant. On peut aussi utiliser le support de l'invention pour extraire ou éliminer un anticorps du sang en fixant sur le support l'antigène correspondant, par exemple par l'intermédiaire des hématies. On peut de même éliminer un substrat d'enzyme du sang en utilisant le support de l'invention sur lequel a été fixée l'enzyme correspondante.

D'autres caractéristiques et avantages de l'invention apparaîtront mieux à la lecture de la description qui suit donnée bien entendu à titre illustratif et non limitatif en référence au dessin annexé sur lequel :

-la figure 1 représente de façon schématique un épurateur plasmatique sur colonne,

-la figure 2 représente un autre type d'épurateur plasmatique sur colonne, et

-la figure 3 représente schématiquement une installation d'épuration sur sang total sur colonne.

Les supports en particules de l'invention sur lesquels ont été fixées des substances biologiques peuvent être utilisés pour l'épuration du sang total ou du plasma sanguin dans les épurateurs représentés sur les figures 1 à 3.

Sur la figure 1, qui illustre l'épuration en continu du plasma sanguin, on voit que l'épurateur comprend une colonne 1 contenant le support de l'invention, un séparateur de cellules 3 pour isoler le plasma sanguin du sang à épurer et un collecteur 5 dans lequel sont introduits, d'une part, les cellules séparées (en 3) du sang à traiter et, d'autre part, le plasma épuré.

Dans cette installation, une première conduite 7 munie d'une pompe 9 et d'un moyen de mesure de la pression 11 sert à introduire le sang provenant du patient dans le séparateur de cellules 3. Dans ce séparateur, les cellules sont évacuées par la conduite 13 vers le collecteur 5 tandis que le plasma est dirigé par la conduite 15 munie d'une pompe 17 dans la colonne 1 contenant le support de l'invention. A la sortie de la colonne 1, le plasma purifié est évacué par la conduite 19 munie d'un moyen de mesure de pression 21 dans le collecteur 5 qui constitue, de plus, un système de sécurité pour éviter la présence de bulles dans le sang ainsi reconstitué. Celui-ci est évacué par la conduite 23 munie du moyen de mesure de pression 25 dans le système circulatoire du patient. Ainsi, les conduites 7 et 23 qui servent respectivement à l'introduction du sang dans le dispositif et à son retour dans le système circulatoire sont montées en dérivation sur une veine du patient.

Sur la figure 2, on a représenté un dispositif d'épuration dans lequel l'épuration du plasma se fait de façon discontinue alors que le système est parcouru en continu par le sang du patient. On retrouve la plupart des constituants du système épurateur de la figure 1 et ils portent les mêmes références. Dans ce cas, le plasma sanguin sortant du séparateur de cellules 3 est purifié en discontinu dans les récipients 31 et 33 contenant le support de l'invention. Dans ce but, le plasma mis en circulation par la pompe 17 peut être extrait du circuit et introduit dans le récipient 31 par la vanne de soutirage 18. Après purification dans le récipient 31, le plasma épuré est réintroduit en amont de la pompe 17 par la conduite 16 alors que la vanne 14 est fermée.

Sur la figure 3, on a représenté un épurateur sur sang total. Dans ce cas, l'installation correspond sensiblement au schéma de la figure 1, mais le séparateur de cellules 3, la conduite 13 et la pompe 17 ont été supprimés puisque l'épuration est réalisée directement sur le sang provenant du patient.

Les exemples suivants sont donnés bien entendu à titre non limitatif pour illustrer la préparation des supports en copolymère de l'invention, la fixation sur ces supports d'une substance biologique et leur utilisation pour l'épuration plasmatique.

## I. EXEMPLES DE PREPARATION DE SUPPORTS

Les exemples 1 à 7 illustrent la préparation de supports en copolymère de méthacrylate de diméthylaminoéthyle et de triméthacrylate de triméthylol propane dans des conditions différentes et l'exemple 8 illustre la préparation d'un support en copolymère d'acroléine et de triméthacrylate de triméthylol propane. Les exemples 1 à 5 et 7 et 8 se réfèrent à une polymérisation en masse, alors que l'exemple 6 concerne la polymérisation en émulsion.

### EXEMPLE 1 :

On prépare un mélange de monomères comprenant 30% en poids de méthacrylate de diméthylaminoéthyle - (MADAM) et 70% en poids de triméthacrylate de triméthylol propane et on désoxygène le mélange en le soumettant à deux dégazages successifs sous vide, puis on l'introduit dans une ampoule et on l'y maintient sous une pression réduite de $5.10^{-2}$ Torr (6,75 Pa). On soumet alors l'ampoule à une irradiation au moyen de rayonnements $\gamma$ issus d'une source de cobalt 60 en utilisant un débit de dose de 0,150 Mrad/h (1500 Gy/h) et en réalisant l'irradiation pendant 17 h.

Après cette irradiation, le produit obtenu est broyé et tamisé à la granulométrie désirée.

### EXEMPLE 2 :

On prépare comme dans l'exemple 1 un mélange de monomères contenant 30% en poids de méthacrylate de diméthylaminoéthyle et 70% en poids de triméthacrylate de triméthylol propane et on le soumet à une irradiation au moyen de rayonnements $\gamma$ en utilisant un débit de dose de 0,029 Mrad/h (290 Gy/h) et en réalisant l'irradiation pendant 110 h.

Après polymérisation, on soumet le produit obtenu à un broyage et on tamise le produit broyé à la granulométrie désirée.

### EXEMPLE 3

On prépare comme dans l'exemple 1 un mélange de monomères contenant 40% en poids de méthacrylate de diméthylaminoéthyle et 60% en poids de triméthacrylate de triméthylol propane. On traite le mélange comme dans l'exemple 1 et on le polymérise dans les mêmes conditions que celles de l'exemple 1. Après irradiation, on soumet également le produit obtenu à un broyage.

### EXEMPLE 4 :

On prépare comme dans l'exemple 1 un mélange de monomères comprenant comme dans l'exemple 1, 30% en poids de méthacrylate de diméthylaminoéthyle et 70% en poids de triméthacrylate de triméthylol propane. On traite le mélange comme dans l'exemple 1 pour le désoxygéner et on le laisse sous une atmosphère inerte d'azote.

On réalise ensuite la polymérisation en utilisant un accélérateur d'électrons d'une énergie de 3 MeV, d'une intensité de 400 $\mu$A en laissant le mélange dans l'accélérateur pendant une durée suffisante pour que la dose intégrée appliquée soit de 4 Mrad.

Après polymérisation, le support est ensuite broyé et tamisé à la granulométrie désirée.

### EXEMPLE 5 :

On utilise le même mode opératoire que dans l'exemple 4 mais avec un mélange de monomères comprenant 80% en poids de méthacrylate de diméthylaminoéthyle et 20% en poids de triméthacrylate de triméthylol propane.

### EXEMPLE 6

Dans cet exemple, on réalise la polymérisation en émulsion. On prépare tout d'abord un mélange de monomères comprenant 30% en poids de méthacrylate de diméthylaminoéthyle et 70% en poids de triméthacrylate de trimèthylol propane et on soumet le mélange à deux dégazages successifs comme dans l'exemple 1. On prépare ensuite l'émulsion dans un réacteur équipé d'un dispositif d'agitation en dispersant le mélange de monomères dans une phase aqueuse saturée en chlorure de sodium, légèrement basique, contenant $7{,}5.10^{-2}$ mol/l de NaOH. Le rapport pondéral de la phase organique sur la phase aqueuse est de 2/3 et la vitesse d'agitation utilisée pour préparer et maintenir les deux phases en émulsion est de 650 tours/min. On réalise ensuite la polymérisation sous atmosphère d'azote dans le réacteur, avec une vitesse

d'agitation de 650 tours/min. en irradiant au moyen de rayonnements $\gamma$ à un débit de dose de 0,130 Mrad/h (1300 Gy/h) pendant 17 h, ce qui correspond à une dose intégrée de 2,2 Mrad (22100 Gy).

On obtient ainsi des particules de copolymère ayant une granulométrie de 40 à 80 $\mu$m.

Les conditions de fabrication et les résultats obtenus dans les exemples 1 à 6 sont récapitulées dans le tableau 1 annexé.

EXEMPLE 7

On prépare comme dans l'exemple 1 un mélange de monomères comprenant 50% en poids de méthacrylate de diméthylaminoéthyle (MADAM) et 50% en poids de triméthacrylate de triméthylol propane. On traite ensuite ce mélange et on le polymérise dans les mêmes conditions que celles de l'exemple 1. Après irradiation, on soumet également le produit obtenu à un broyage.

EXEMPLE 8

On prépare un mélange de monomères comprenant 70% en poids d'acroléine et 30% en poids de triméthacrylate de triméthylol propane (TMATMP). On le polymérise ensuite en masse sous vide par irradiation au moyen de rayonnements gamma en utilisant un débit de dose de 0,048 Mrad/h et en réalisant l'irradiation pendant une durée telle que la dose intégrée appliquée soit de 7,7 Mrad.

Après cette irradiation, le produit obtenu est broyé et tamisé à la granulométrie désirée.

II. FIXATION DE SUBSTANCES BIOLOGIQUES

Les exemples 9 à 11 illustrent la fixation d'hématies sur les supports particulaires des exemples 1 à 7. La fixation d'hématies permet de réaliser une épuration spécifique d'anticorps anti-D humains qui est d'un grand intérêt pour le traitement des incompatibilités foetomaternelles Rhésus.

En effet, les anticorps de groupe sanguin ont des spécificités divisées contre les déterminants antigéniques portés à la surface des hématies, ce qui permet de les extraire après fixation des hématies sur un support solide.

L'exemple 12 illustre la fixation d'héparine qui présente la propriété de former des complexes stables avec les lipoprotéines de faible densité associées au cholestérol. Ces complexes se forment par l'intermédiaire de liaisons ioniques entre les groupes chargés négativement des polysaccharides ($SO_3^-$, $COO^-$, $NHSO_3^-$), et les groupements positifs de la partie protéique des lipoprotéines - ($NH_2^+$).

L'exemple 13 illustre la fixation d'acide $\epsilon$-aminocaproïque sur le support de l'exemple 8.

Les exemples 14 et 15 illustrent la fixation d'$\alpha$-chymotrypsine sur le support de l'exemple 8.

Les exemples 16 et 17 illustrent la fixation de $\gamma$-globulines sur le support de l'exemple 8.

EXEMPLE 9 : Fixation d'hématies

On utilise les supports en copolymère obtenus dans les exemples 1, 2, 3, 4 et 6, après avoir isolé les fractions ayant une granulométrie de 40 à 80 $\mu$m.

On met en suspension les particules de support dans une solution tampon constituée par une solution de saccharose-PBS (tampon au phosphate) ayant des concentrations respectives en saccharose et en PBS de 7 et de 3, les concentrations cumulées de ces deux composés étant ajustées pour obtenir une osmolarité de 310 mOSM, en utilisant un rapport en volume de 1 entre les particules et la solution. On ajoute cette suspension goutte à goutte à une suspension ayant également un rapport en volume de 1 de globules rouges dans la même solution tampon en utilisant des quantités de suspension telles qu'elles correspondent à 1 volume de particules pour 0,4 volume de globules rouges. On soumet les suspensions à une agitation puis on laisse décanter les particules et on élimine

On prépare alors une solution de glutaraldéhyde dans la même solution tampon saccharose-PBS à 310 m OSM et pH 6, et on met les particules en suspension dans cette solution de glutaraldéhyde à raison d'un volume de particules pour 5 volumes de solution de glutaraldéhyde. La concentration finale de glutaraldéhyde est de 0,12%. On laisse la réaction se poursuivre pendant 20 min., à la température ambiante, sous une agitation rotative très lente. On sépare alors les particules de la solution et on les soumet à différents rinçages effectués à 40°C dans du sérum physiologique, puis à une incubation pendant une heure dans une solution à 0,1 mol/l de glycine dans le tampon PBS à 4°C et à un rinçage final au moyen du tampon PBS.

Dans ces conditions, la quantité d'hématies fixées sur les particules est d'environ 25000 hématies par mm², ce qui correspond au recouvrement de la surface des particules par une monocouche d'hématies.

EXEMPLE 10 : fixation d'hématies.

On utilise le même mode opératoire que dans l'exemple 9, mais en utilisant le support en particules obtenu dans l'exemple 5, après avoir isolé la fraction ayant une granulométrie de 40 à 80 $\mu$m. On obtient ainsi un support sur lequel sont fixées environ 15000 hématies par mm².

EXEMPLE 11 : Fixation d'hématies.

On utilise le même mode opératoire que dans l'exemple 9, mais en utilisant le support en particules obtenu dans l'exemple 7, après avoir isolé la fraction ayant une granulométrie de 40 à 80 $\mu$m. On obtient ainsi un support sur lequel sont fixées au moins 50000 hématies par mm².

EXEMPLE 12 : Fixation d'héparine.

Dans cet exemple, on utilise le support obtenu dans l'exemple 5, après avoir isolé la fraction de particules ayant une granulométrie de 160 à 315 $\mu$m.

On lave tout d'abord les particules de support dans une solution d'acide chlorhydrique 1N, puis on met en suspension 3 ml de particules dans 5 ml d'une solution d'héparine à 1000 u dans NaCl 0,1M à pH 3, et l'on maintient la suspension sous agitation rotative pendant 1h30 à 65°C. On sépare ensuite les particules et on les rince avec du sérum physiologique.

On prépare ensuite une solution de glutaraldéhyde dans l'eau contenant 0,06% de glutaraldéhyde à pH 7. On met alors les particules en suspension dans la solution de glutaraldéhyde à raison d'un volume de particules pour 5 volumes de solution et on les maintient dans la solution pendant une heure à la température ambiante sous agitation rotative.

On sépare ensuite les particules de la solution et on les soumet à différents rinçages au moyen de sérum physiologique.

EXEMPLE 13 : Fixation d'acide ε-aminocaproïque

Dans cet exemple, on utilise le support en copolymère obtenu dans l'exemple 8 après avoir isolé la fraction ayant une granulométrie de 100 à 200 μm, et on fixe sur celui-ci de l'acide ε-aminocaproïque selon le schéma réactionnel suivant :

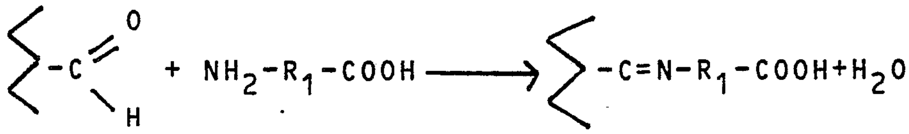

en utilisant pour le couplage une solution à 10% d'acide ε-aminocaproïque dans le tampon acétate M/10, pH 5,3 contenant 1% de dodécylsulfate de sodium

On met en contact 100 g de copolymère avec 1 l de cette solution, à une température de 37°C, pendant 2 h dans un bain-marie avec une agitation de va-et-vient. En fin de réaction, on lave le copolymère dans une solution tampon acétate M/10, pH 5,3, contenant 1% de dodécylsulfate de sodium, à 37°C, pendant 24 h, puis dans la même solution tampon contenant 0,06% de dodécylsulfate de sodium à 37°C, pendant 24 h. On détermine ensuite la quantité d'acide ε-aminocaproïque fixé par dosage en retour des fonctions -COOH en bout de chaîne au moyen de NaOH. Dans ces conditions, on trouve que la quantité d'acide ε-aminocaproïque fixé est de 32,1 g pour 100 g de copolymère, ce qui correspond à 19,6 mol d'acide ε-aminocaproïque pour 100 mol d'acroléine.

EXEMPLE 14 : Fixation d'α-chymotrypsine

Dans cet exemple, on utilise le support en copolymère de l'exemple 8 après avoir isolé la fraction ayant une granulométrie de 100 à 200 μm.

La réaction de couplage avec l'α-chymotrypsine ($NH_2$-αC) correspond au schéma réactionnel suivant :

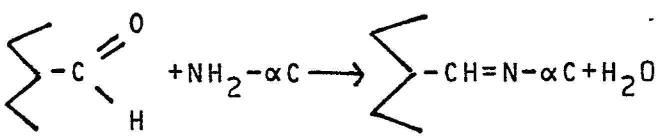

Pour cette réaction de couplage, on utilise une solution d'α-chymotrypsine à 2 g/l dans le tampon MES 0,1M, pH 7, et l'on met en contact 100 g de copolymère avec 1 l de cette solution, pendant 5 H, à 5°C, sous agitation magnétique. Après cette réaction, on soumet le copolymère à des lavages progressifs à 5°C pendant au moins 7 h, en utilisant successivement les solutions suivantes de forces ioniques différentes :

-tampon MES 0,1M, pH 7,

-tampon MES 0,1M, NaCl 1M, pH 7,

-HCl $10^{-3}$M, NaCl 1M, pH 3, et

-Tris 0,01M, $CoCl_2$ 0,05M, pH 8.

On détermine ensuite la quantité d'α-chymotrypsine fixée par mesure de l'activité enzymatique. Cette mesure est effectuée à partir de l'hydrolyse de l'ester éthylique de l'acétyltyrosine (ATEE) par l'enzyme, qui est suivie par la consommation de NaOH pour maintenir le pH constant.

On réalise l'hydrolyse dans une solution tampon Tris 0,05M, KCl 0,05M, $CoCl_2$ 0,01M à un pH de 8 pour l'α-chymotrypsine libre et à un pH de 11 pour l'α-chymotrypsine liée.

On trouve ainsi que l'activité de l'α-chymotrypsine fixée sur le polymère est de 763 μmol/$OH^-$/min/g.

EXEMPLE 15 : Fixation d'α-chymotrypsine

Dans cet exemple, on utilise le support en copolymère obtenu dans l'exemple 8, après avoir isolé la fraction ayant une granulométrie de 100 à 200 μm. La réaction de couplage avec l'α-chymotrypsine est effectuée après traitement du copolymère par le bisulfite de sodium qui est un agent d'hydrophilisation. Ce traitement consiste à mettre en contact 100 g de copolymère avec 0,8 l d'une solution de bisulfite de sodium à 107,5 g/l à pH 5,3, pendant 5 h à 65°C, sous balayage d'azote.

Après cette opération, on réalise le couplage de l'α-chymotrypsine dans les mêmes conditions que celles de l'exemple 14.

Dans ces conditions, l'activité de l'α-chymotrypsine fixée sur le polymère est de 1026 μmol/$OH^-$/min/g.

EXEMPLE 16 : Fixation de γ-globulines

Dans cet exemple, on utilise le support en copolymère obtenu dans l'exemple 8 après avoir isolé la fraction ayant une granulométrie de 100 à 200 μm.

La réaction de couplage avec les γ-globulines ($NH_2^-$γG) correspond au schéma réactionnel suivant :

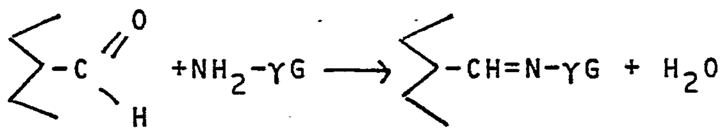

et elle est effectuée dans les mêmes conditions que celles de l'exemple 13, en remplaçant l'acide ε-aminocaproïque par des γ-globulines marquées à l'iode 125 et en utilisant une concentration en γ-globulines de la solution de 1 g/l.

On détermine ensuite la quantité de γ-globulines fixées par comptage de la radioactivité et l'on trouve ainsi que la quantité de γ-globulines fixées est de 0,32 mg/g de copolymère.

EXEMPLE 17 : Fixation de γ-globulines

On utilise le support de l'exemple 8 après avoir isolé la fraction ayant une granulométrie de 100 à 200 μm. On réalise la fixation de γ-globulines marquées à l'iode 125 après avoir couplé à ce support de l'acide ε-aminocaproïque en utilisant le mode opératoire de l'exemple 13. On active alors les fonctions carboxyliques ainsi fixées sur le support par la carbodiimide selon le schéma réactionnel suivant :

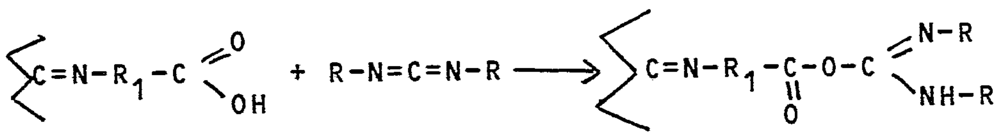

Pour réaliser cette activation, on utilise une solution de carbodiimide à 50 mg/ml dans du tampon morpholinoéthylsulfonate (MES) 0,1 M, pH 4,75, et l'on met en contact 100 g de copolymère avec 0,2 l de solution pendant une heure à la température ambiante. On effectue ensuite plusieurs lavages rapides du copolymère dans le tampon MES 0,1 M, pH 4,75. On fixe ensuite sur le support des gamma-globulines marquées à l'iode 125 en utilisant une solution contenant 1 mg/ml de gamma-globulines dans le tampon MES 0,1 M, pH 4,75 contenant 1% de Tween 20 et on met en contact 100 g du copolymère ayant subi le traitement d'activation avec 1 l de solution pendant 17 h à une température de 4°C. Après la réaction, on soumet le copolymère à plusieurs lavages dans le tampon MES 0,1 M, pH 4,75.

On détermine ensuite la quantité de gamma-globulines marquées fixées par mesure de la radioactivité. On trouve ainsi que la quantité de gamma-globulines fixées est de 0,40 mg/g de support.

III. UTILISATION DES SUPPORTS POUR L'EPURATION PLASMATIQUE

EXEMPLES 18 à 20 : Epuration "in vitro" d'anticorps anti-D humain.

Dans ces exemples, on utilise le support en particules obtenu dans l'exemple 9 pour épurer des plasmas citratés contenant des anticorps anti-D humain qui ont été prélevés sur différents malades.

On dose tout d'abord les anticorps dans chacun des plasmas en utilisant une méthode semi-quantitative d'agglutination, puis on met en contact les plasmas avec le support en particules, pendant une heure, à une température de 37°C, sous une agitation rotative, en utilisant différentes proportions de support par rapport au volume de plasma. En fin d'opération, on dose les quantités d'anticorps restant dans les plasmas en utilisant la même méthode semi-quantitative d'agglutination. Les résultats obtenus sont donnés dans le tableau 2 qui suit.

TABLEAU 2

| EXEMPLES | 18 | 19 | 20 |
| --- | --- | --- | --- |
| rapport $\frac{\text{volume particules}}{\text{volume plasma}}$ | 1 | 0,5 | 0,5 |
| Concentration initiale du plasma en anticorps anti-D (μg/ml) | 11,7 | 11,7 | 2,5 |
| Taux d'anticorps anti-D épuré (%) | 48 | 38 | 75 |

Au vu de ce tableau, on voit que les supports de l'invention sur lesquels ont été fixées des hématies permettent d'obtenir des taux d'épuration élevés en anticorps anti-D humain.

EXEMPLE 21 : Epuration "in vitro" d'anticorps anti-D humain.

Dans cet exemple, on utilise le support en particules sur lesquelles sont fixées les hématies, obtenu dans l'exemple 10 pour épurer des anticorps anti-D humain présents dans du plasma citraté prélevé sur un malade dont la concentration initiale en anticorps anti-D est de 2,5 µg/ml de plasma.

On réalise l'épuration dans les mêmes conditions que celles de l'exemple 18 en utilisant un rapport volume de particules/volume de plasma égal à 0,5.

Dans ces conditions, on obtient un taux d'épuration d'anticorps anti-D humain de 52%.

EXEMPLE 22 : Epuration "in vitro" d'anticorps anti-D humains.

Dans cet exemple, on utilise le support en particules sur lesquelles sont fixées les hématies, obtenu dans l'exemple 11 pour épurer des anticorps anti-D humains présents dans du plasma citraté prélevé sur un malade dont la concentration initiale en anticorps anti-D est de 2,5 µg/ml de plasma.

On réalise l'épuration dans les mêmes conditions que celles de l'exemple 18.

Cependant dans ces conditions, l'épuration est impossible car la quantité d'hématies fixée sur les particules de copolymère est trop importante, et il se produit un relargage d'hématies dans le plasma. Ainsi, la formulation du support en copolymère de l'exemple 7 ne convient pas pour la fixation d'hématies car une partie de celles-ci ne sont pas fixées de façon irréversible sur le support.

EXEMPLE 23 : Epuration plasmatique "in vitro" des lipoprotéines de faible densité associées au cholestérol.

Dans cet exemple, on utilise le support en particules obtenu dans l'exemple 12 sur lequel est fixée de l'héparine, pour épurer les lipoprotéines de faible densité présentes dans un plasma prélevé sur un malade. On dose tout d'abord le cholestérol du plasma par une méthode colorimétrique après hydrolyse enzymatique, puis on met en contact le plasma avec le support en particules à raison d'un volume de support pour deux volumes de plasma, pendant 10 min, à une température de 37°C, sous agitation rotative. On détermine en fin d'opération la concentration en cholestérol du plasma et l'on trouve ainsi que pour une concentration initiale de 2,68 g/l de cholestérol dans le plasma, la quantité de cholestérol épurée est de 0,88 mg/ml de support en particules, ce qui correspond à un taux d'épuration de 16 %.

TABLEAU 1

| EXEMPLES | MADAM* (%) | TMATMP* (%) | Débit de dose (Gy/h) | Dose intégrée (Gy) |
|---|---|---|---|---|
| EX. 1 | 30 | 70 | 1 500 | 25 500 |
| EX. 2 | 30 | 70 | 290 | 31 900 |
| EX. 3 | 40 | 60 | 1 500 | 25 500 |
| EX. 4 | 30 | 70 | | 40 000 |
| EX. 5 | 80 | 20 | | 40 000 |
| EX. 6 | 30 | 70 | 1 300 | 22 100 |
| EX. 7 | 50 | 50 | 1 500 | 25 500 |

* MADAM : méthacrylate de diméthylaminoéthyle

TMATMP : triméthacrylate de triméthylol propane.

## Revendications

1. Support solide capable de fixer de façon irréversible une substance biologique, caractérisé en ce qu'il est constitué par un copolymère :

-d'au moins un monomère choisi parmi les monomères acryliques, les monomères méthacryliques et les monomères allyliques, porteurs d'au moins une fonction chimique réactive présentant une affinité pour la substance biologique à fixer, choisie parmi les fonctions amine primaire, amine secondaire, amine tertiaire, hydroxyle, aldéhyde et amide, et

-d'au moins un monomère réticulant comportant au moins deux fonctions éthyléniques réactives sous rayonnements ionisants.

2. Support selon la revendication 1, caractérisé en ce qu'il comprend :

-30 à 80% en poids dudit ou desdits monomères porteurs d'au moins une fonction chimique réactive présentant une affinité pour la substance biologique à fixer, et

-de 20 à 70% en poids dudit ou desdits monomères réticulants.

3. Support selon l'une quelconque des revendications 1 et 2, caractérisé en ce que le monomère porteur d'au moins une fonction chimique présentant une affinité pour la substance biologique à fixer est un acrylate ou un méthacrylate de dialkylaminoalkyle.

4. Support selon la revendication 3, caractérisé en ce que le monomère porteur d'au moins une fonction chimique présentant une affinité pour la substance biologique à fixer est le méthacrylate de diméthylaminoéthyle.

5. Support selon l'une quelconque des revendications 1 et 2, caractérisé en ce que le monomère porteur d'au moins une fonction chimique présentant une affinité pour la substance biologique à fixer est l'acroléine.

6. Support selon l'une quelconque des revendications 1 et 2, caractérisé en ce que le monomère porteur d'au moins une fonction chimique présentant une affinité pour la substance biologique à fixer est l'acrylate d'éthylène glycol.

7. Support selon l'une quelconque des revendications 1 à 6, caractérisé en ce que le monomère réticulant est un polyacrylate ou un polyméthacrylate de polyol.

8. Support selon la revendication 7, caractérisé en ce que le monomère réticulant est choisi parmi le diacrylate d'éthylène glycol, le diacrylate de tétraéthylène glycol, le triacrylate de triméthylol propane, le diméthacrylate d'éthylène glycol, le diméthacrylate de tétraéthylène glycol, le triméthacrylate de triméthylol propane, le tétracrylate de pentaérythrol et le tétraméthacrylate de pentaérythrol.

9. Support selon l'une quelconque des revendications 1 et 2, caractérisé en ce qu'il est constitué par un copolymère de méthacrylate de diméthylaminoéthyle et de triméthacrylate de triméthylol propane.

10. Procédé de préparation d'un support selon l'une quelconque des revendications 1 à 9, caractérisé en ce qu'il consiste à polymériser au moyen de rayonnements ionisants un mélange de monomères comprenant au moins un monomère choisi parmi les monomères acryliques méthacryliques et allyliques, porteurs d'au moins une fonction chimique présentant une affinité pour la substance biologique à fixer et au moins un monomère réticulant comportant au moins deux fonctions éthyléniques réactives sous rayonnements ionisants.

11. Procédé selon la revendication 10, caractérisé en ce que les rayonnements ionisants sont choisis parmi les faisceaux d'électrons et les rayonnements $\gamma$.

12. Procédé selon l'une quelconque des revendications 10 et 11, caractérisé en ce que l'on réalise la polymérisation en masse et en ce que l'on soumet ensuite le copolymère obtenu à un broyage et à un tamisage.

13. Procédé selon l'une quelconque des revendications 10 et 11, caractérisé en ce que l'on réalise la polymérisation en émulsion.

14. Support contenant une substance biologique, caractérisé en ce qu'il est constitué par des particules d'un support solide selon l'une quelconque des revendications 1 à 9 sur lequel est fixée une substance biologique.

15. Support selon la revendication 14, caractérisé en ce que le support solide est constitué par un copolymère d'au moins un monomère choisi parmi les monomères acryliques, méthacryliques et allyliques porteurs d'au moins une fonction choisie parmi les fonctions amine primaire, amine secondaire et amine tertiaire, et d'au moins un monomère réticulant comportant au moins deux fonctions éthyléniques réactives sous rayonnements ionisants, et en ce que la substance biologique est constituée par des hématies.

16. Support selon la revendication 14, caractérisé en ce que le support solide est constitué par un copolymère d'au moins un monomère choisi parmi les monomères acryliques, les monomères méthacryliques et les monomères allyliques, porteurs d'au moins une fonction choisie parmi les fonctions amine primaire, amine secondaire et amine tertiaire, et d'au moins un monomère réticulant comportant au moins deux fonctions éthyléniques réactives sous rayonnements ionisants et en ce que la substance biologique est constituée par de l'héparine.

17. Procédé de préparation d'un support selon la revendication 14, caractérisé en ce qu'il consiste à mettre en contact les particules d'un support solide selon l'une quelconque des revendications 1 à 7 avec une substance biologique en solution ou en suspension et à soumettre ensuite le support séparé de la solution ou de la suspension et ayant absorbé ladite substance à l'action d'un réactif capable de fixer ladite substance sur le support.

18. Procédé selon la revendication 17, caractérisé en ce que le réactif est le glutaraldéhyde.

19. Procédé selon la revendication 17, caractérisé en ce que le réactif est le carbodiimide.

20. Utilisation du support selon la revendication 14 pour extraire ou éliminer un élément biologique du sang, caractérisée en ce que la substance biologique fixée sur ledit support est une substance capable de réagir avec ledit élément.

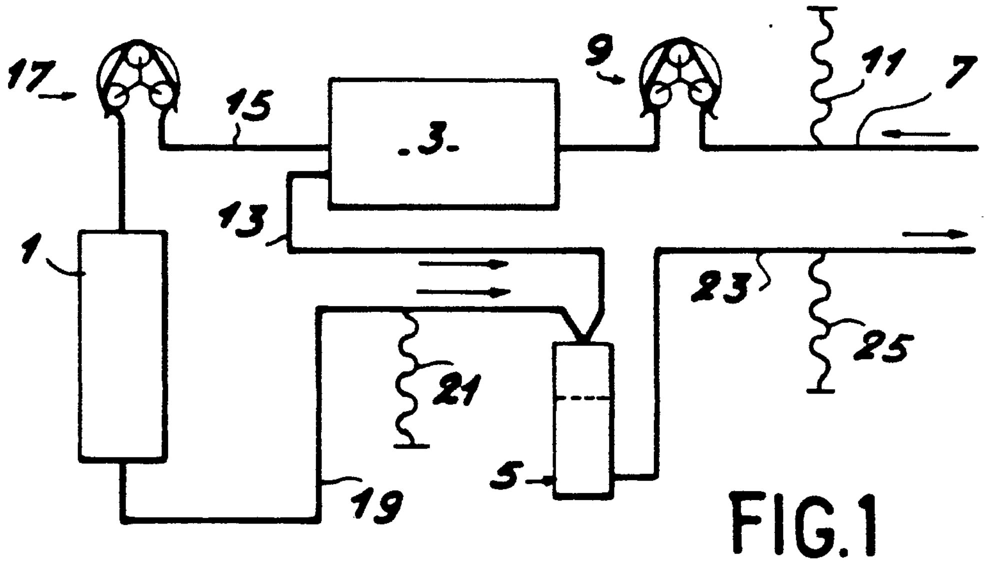


FIG.1

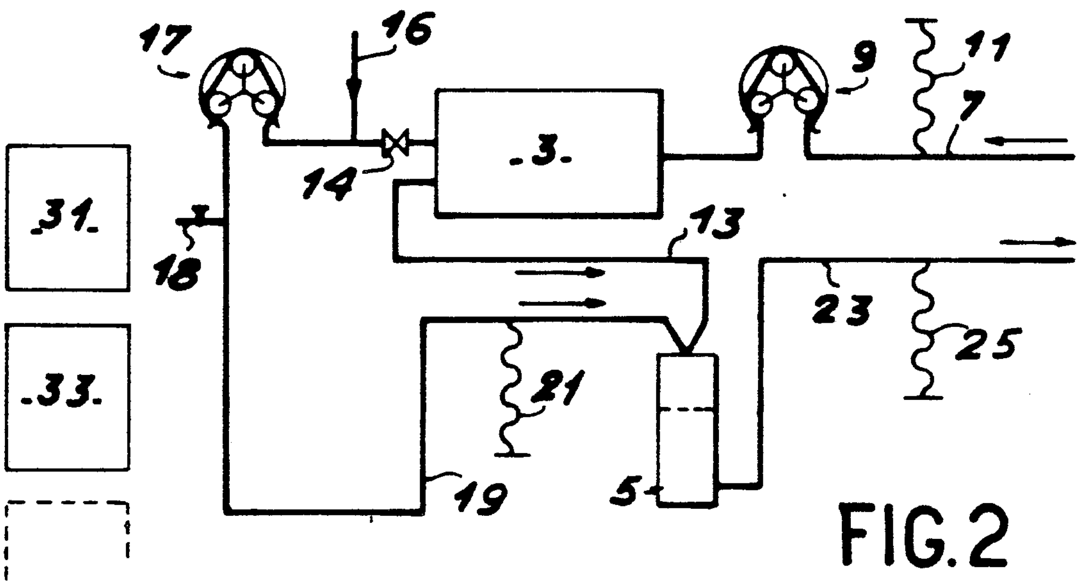


FIG.2

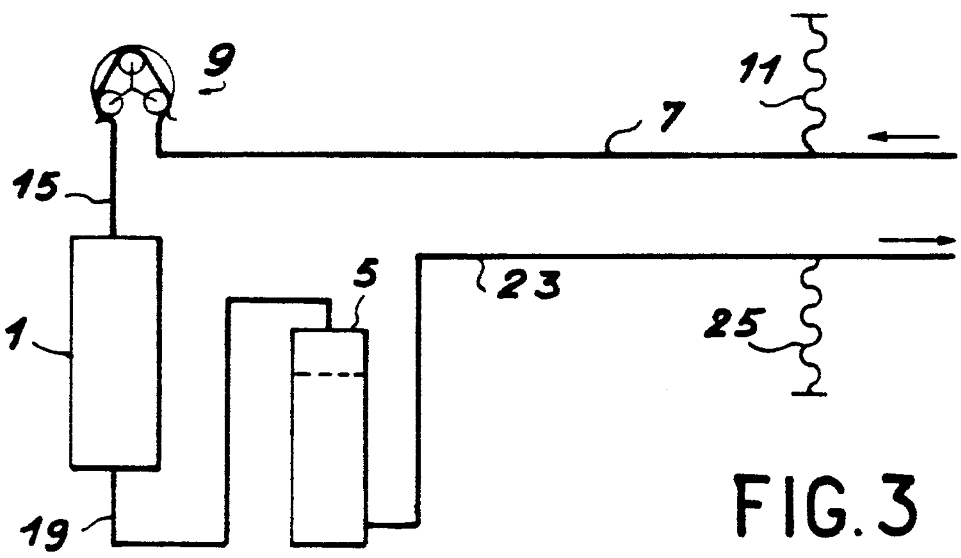


FIG.3

Office européen des brevets

# RAPPORT DE RECHERCHE EUROPEENNE

Numéro de la demande

EP 86 40 0422

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-4 105 598 (S.P.S. YEN et al.)<br>* Revendication 1 *<br>--- | 1-19 | C 08 F 220/28<br>C 08 F 220/34<br>B 01 J 20/26<br>C 07 K 17/08 |
| X | EP-A-0 063 947 (SEKISUI KAGAKU K.K.)<br>* Revendication 1 *<br>----- | 1-19 | |

| DOMAINES TECHNIQUES RECHERCHES (Int. Cl.4) |
|---|
| C 08 F<br>B 01 J |

Le présent rapport de recherche a été établi pour toutes les revendications

| Lieu de la recherche | Date d'achèvement de la recherche | Examinateur |
|---|---|---|
| LA HAYE | 10-06-1986 | CAUWENBERG C.L.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant